(19) **Europäisches Patentamt**
**European Patent Office**
**Office européen des brevets**

(11) **EP 1 106 983 B1**

(12) **EUROPEAN PATENT SPECIFICATION**

(45) Date of publication and mention
of the grant of the patent:
**30.09.2009 Bulletin 2009/40**

(51) Int Cl.:
**G01N 1/22** (2006.01)

(21) Application number: **00126433.2**

(22) Date of filing: **06.12.2000**

(54) **Exhaust gas analyzing system**

System zur Abgasanalyse

Système d'analyse de gaz d'échappement

(84) Designated Contracting States:
**DE FR GB**

(30) Priority: **06.12.1999 JP 34585499**

(43) Date of publication of application:
**13.06.2001 Bulletin 2001/24**

(73) Proprietor: **HORIBA, LTD.**
**Kyoto (JP)**

(72) Inventors:
• **Inoue, Kaori**
**Minami-ku,**
**Kyoto (JP)**

• **Adachi, Masayuki**
**Minami-ku,**
**Kyoto (JP)**

(74) Representative: **Müller - Hoffmann & Partner**
**Patentanwälte**
**Innere Wiener Strasse 17**
**81667 München (DE)**

(56) References cited:
**EP-A- 0 855 578**   **US-A- 4 928 015**
**US-A- 5 650 565**   **US-A- 5 968 452**

• **PATENT ABSTRACTS OF JAPAN vol. 2000, no. 03, 30 March 2000 (2000-03-30) & JP 11 344425 A (HORIBA LTD), 14 December 1999 (1999-12-14)**

**Description**

The present invention relates to an exhaust gas analyzing system.

A known CVS method (Constant Volume Sampling) that is currently used widely as a sampling method of measuring mass of components in gas exhausted from an engine of an automobile, for example, is prone to insufficient accuracy when measuring the exhaust gas of a ULEV (Ultra Low Emission Vehicle), a SULEV (Super Low Emission Vehicle), and the like.

As substitute for the above CVS method, a mini-diluter method has been proposed. In this mini-diluter method, instead of diluting the whole quantity of the exhaust gas from the engine, a part thereof is sampled, the sampled exhaust gas part is diluted at a certain dilution ratio, the diluted sample gas is gathered in a sample bag by an amount proportional to a flow rate of the exhaust gas from the engine, and then the diluted sample gas in the sample bag is analyzed.

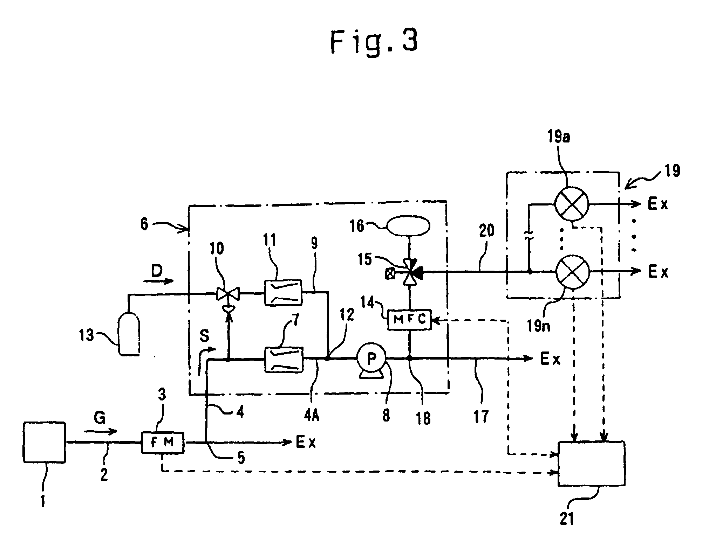

Fig. 3 schematically shows an example of an arrangement of an exhaust gas analyzing system for which the mini-diluter method is used. Reference numeral 1 designates an engine of an automobile, 2 an exhaust gas flow path connected to an exhaust pipe connected to the engine 1, and 3 a flowmeter (digital flowmeter, for example) for measuring a flow rate of the whole exhaust gas G flowing through the exhaust gas flow path 2. Reference numeral 4 designates a sampling flow path that is connected to the exhaust gas flow path 2 at a point 5 downstream from the flowmeter 3 and samples a part of the exhaust gas G flowing through the exhaust gas flow path 2 as sample gas S.

Reference numeral 6 designates a mini-diluter arranged in the sampling flow path 4 having the following components: Reference numeral 4A designates a sampling flow path in the mini-diluter 6 in which a CFV (critical flow venturi) 7 for defining a flow rate of the sample gas S flowing through the sampling flow path 4A followed by a suction pump 8 are provided. Reference numeral 9 designates a dilution gas flow path provided in parallel with the sampling flow path 4A in which a pressure controller 10 and then a CFV 11 for defining a flow rate of the dilution gas D flowing through the dilution gas flow path 9 are provided. The downstream side of the CFV 11 is connected to the CFV 7 of the sampling flow path 4A at a point 12 between the CFV 7 and the pump 8. The pressure controller 10 has the function of equalizing the pressure on an inlet side of the CFV 7 of the flow path 4A with pressure on an inlet side of the CFV 11 of the dilution gas flow path 9. On the upstream side of the pressure controller 10 (more specifically, on an outside of the mini-diluter 6), a cylinder 13 containing dilution gas (e. g., nitrogen gas) is provided.

On the downstream side of the sampling flow path 4A from the suction pump 8, a sample bag 16 is provided through a mass-flow controller 14 (MFC) that is formed of a flow rate measuring portion and a flow rate control valve and has both functions of measuring and controlling the flow rate by use of a three-way solenoid valve 15 as a selector valve. Reference numeral 17 designates an overflow flow path connected to a point 18 between the suction pump 8 of the sampling flow path 4A and the mass-flow controller 14.

Reference numeral 19 designates a gas analyzing portion provided at the rear stage of the mini-diluter 6. A plurality of gas analyzers 19a to 19n, for example, are provided in parallel with each other in a flow path 20 connected to the three-way solenoid valve 15. For example, the gas analyzers 19a to 19n, are NDIRs (non-dispersive infrared analyzer) for measuring CO and $CO_2$, CLDs (chemiluminescent analyzer) for measuring $NO_x$, FIDs (flame ionization detector) for measuring THC (total hydrocarbon), and the like.

Furthermore, reference numeral 21 designates an arithmetic controller, for example, a personal computer, that performs arithmetic operations based on output signals from the flowmeter 3, mass-flow controller 14, and gas analyzing portion 19, and controls the whole exhaust gas analyzing system based on a specific result of the arithmetic operations.

In the exhaust gas analyzing system having the above structure and for which the mini-diluter method is used, the exhaust gas analysis is carried out as follows: A flow rate of the exhaust gas G from the engine 1 is measured by the flowmeter 3 connected to the exhaust gas flow path 2 and output from the flowmeter 3 is input into the arithmetic controller 21. Because the suction pump 8 in the mini-diluter 6 is operating, a part of the exhaust gas G whose flow rate has been measured is taken in the sampling flow path 4 as the sample gas S. The sample gas S flows through the flow path 4A in the mini-diluter 6 connected to the sampling flow path 4 toward the suction pump 8. By operation of the suction pump 8, the dilution gas D flows through the dilution gas flow path 9 provided in parallel with the flow path 4A.

Because the pressure controller 10 equalizing the pressure on the inlet side of the CFV 7 of the flow path 4A with the pressure on the inlet side of the CFV 11 of the dilution gas flow path 9 is connected with the dilution gas flow path 9 and the CFVs 7 and 11 for defining the flow rates of the gas S and D flowing through the flow paths 4A and 9 are respectively connected with the flow path 4A and the dilution gas flow path 9, ways of changing of the flow rates of the gas S and D flowing through both the flow paths 4A and 9 are equalized with each other and a ratio between the flow rates is always constant. The gas flows S and D merge with each other at a confluence 12 and the sample gas S is diluted with the dilution gas D to a certain consistency.

The diluted sample gas S flows through the suction pump 8 to the downstream thereof and a part of the gas S flows towards the three-way solenoid valve 15, whereby a flow rate of the part of the gas S is determined by the mass-flow controller 14 associated with the flow path 4A. Because the three-way solenoid valve 15 allows the communication between the mass-flow controller 14 and the sample bag 16 when the power is turned off, the diluted sample gas S that has passed through the mass-flow controller 14 is gathered in the sample bag 16. Then, the remainder of the diluted sample gas S is exhausted through the overflow flow path 17.

An opening degree of the flow rate control valve of the mass-flow controller 14 is controlled actively such that the flow rate of the diluted sample gas S taken in by the mass-flow controller 14 is proportional to a flow rate of the exhaust gas G flowing through the exhaust gas flow path 2. More specifically, because the flow rate of the exhaust gas is measured by the flowmeter 3 and the result of the measurement is input into the arithmetic controller 21 as described already, the arithmetic controller 21 sends a control command to set the opening degree of the flow rate control valve and the mass-flow controller 14 takes in a proportional flow rate of the sample gas S to the flow rate of the exhaust gas G.

When the predetermined sampling ends, the power to the three-way solenoid valve 15 is turned on, the sample bag 16 and the flow path 20 communicate with each other, the diluted sample gas S taken into the sample bag 16 is supplied to the gas analyzing portion 19, and concentrations of components to be measured contained in the diluted sample gas S, e. g., CO, $CO_2$, $NO_x$, and THC are respectively measured by the NDIR, CLD, FID, and the like provided in the gas analyzing portion 19.

In this case, mass $M_x$ of a component X before dilution is given by the following equation (1).

$$M_x = C_{xbag} \times V_{ex} \times R_d \times \rho_x \qquad (1)$$

Where $C_{xbag}$ represents a measured concentration of the component X in the bag, $V_{ex}$ the total volume of the exhaust gas, $R_d$ a dilution rate, $\rho_x$ the density of the component X.

As is clear from the above description, because measured concentration $C_{xbag}$ of the concentration of the component X in the bag, the total volume $V_{ex}$ of the exhaust gas, the dilution rate $R_d$, and the density $\rho_x$ of the component X are known, respectively, the mass $M_x$ of the component X in the exhaust gas G before dilution can be obtained easily. According to the exhaust gas analyzing system for which the mini-diluter method is used, the mass of the low-concentration exhaust gas component can be measured with accuracy.

In the exhaust gas analyzing system for which the mini-diluter method is used, however, as is clear from the above description, it is essential to measure the flow rate of the exhaust gas G in real time and a large error may be included in the finally obtained mass of the component in the exhaust gas depending on a degree of a measurement error of the flow rate.

The present invention has been accomplished with the above outlined problem in view and it is an object of the invention to provide an exhaust gas analyzing system in which measurement accuracy of the exhaust gas analyzing system when the mini-diluter method is used can be evaluated by the system itself.

US-Patent No. 5,968,542 discloses an exhaust emission sampling system using a mini-diluter device.

US-Patent No. 4,928,015 discloses an apparatus and method for carrying out constant volume sampling of a gas stream, wherein a tracer gas of known flow rate may be introduced into the gas stream. A gas species to be measured is ratioed to the tracer component concentration after diluting a sample of the gas stream.

This object is used by a method and system according to claims 1 and 2. It is provided an exhaust gas analyzing system, a sampling flow path being connected to an exhaust gas flow path through which gas exhausted from an engine flows to sample a part of the exhaust gas, the sampled exhaust gas being diluted with dilution gas introduced through a dilution gas flow path connected in parallel to the sampling flow path, a part of the diluted exhaust gas being reserved in a sample bag, and the diluted exhaust gas in the sample bag being analyzed in a gas analyzing portion, wherein a flow rate of the exhaust gas is measured, trace gas with a known concentration is introduced into the exhaust gas flow path on an upstream side from a connecting point between the exhaust gas flow path and the sampling flow path while monitoring a flow rate of the trace gas, diluted trace gas is analyzed in the gas analyzing portion, and the total mass of the trace gas calculated from a result of the analysis is compared with the total mass of the introduced trace gas to evaluate the measurement itself.

In the exhaust gas analyzing system, the total mass of the trace gas introduced into the exhaust gas flow is known, and by comparing the total mass of the trace gas measured and calculated by using the mini-diluter method with the above known total mass it is possible to evaluate the accuracy of the measurement itself. As a result, reliability of the exhaust gas analyzing system for which the mini-diluter method is used and which includes measurement of the flow rate of the exhaust gas is improved.

**Fig. 1**    schematically shows an example of a structure of an exhaust gas analyzing system of the present invention.

**Fig. 2**    schematically shows another example of the structure of the exhaust gas analyzing system of the invention.

**Fig. 3**    schematically shows an example of a structure of an exhaust gas analyzing system for which a mini-diluter method is used.

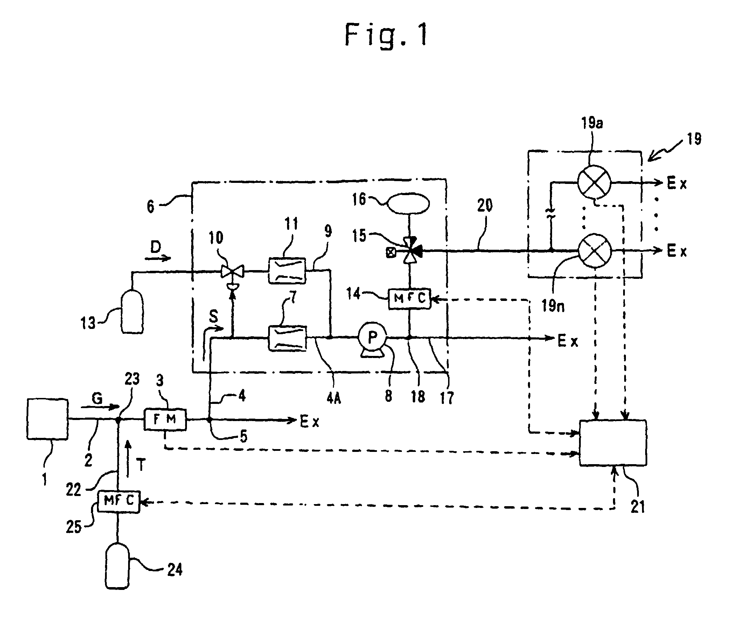

Embodiments of the present invention will be described by reference to the drawings. Fig. 1 shows a first embodiment of an exhaust gas analyzing system of the invention. The exhaust gas analyzing system is significantly different from the prior-art exhaust gas analyzing system in that proper trace gas T with a known concentration and a known flow rate is introduced on an upstream side from a point 5 that is for sampling gas from an exhaust gas flow path 2 into a mini-diluter 6, diluted trace gas T is analyzed by a trace gas analyzer provided in a gas analyzing portion 19, and the total mass of the trace gas T calculated from a result of the analysis is compared with the total mass of the above introduced trace gas T to evaluate the measurement itself.

In Fig. 1, a reference numeral 22 designates a trace gas introducing path for introducing the trace gas T into the exhaust gas flow path 2 and is connected to the exhaust gas flow path 2 at a point 23. On an upstream side of the trace gas introducing path 22, a cylinder 24 of He (helium gas) as the trace gas T and a mass-flow controller 25 for measuring and controlling a flow rate of the trace gas T are provided. The mass-flow controller 25 is controlled by an arithmetic controller 21 and outputs a detected flow rate measurement to the arithmetic controller 21.

In order to analyze the concentration of He as the trace gas, the gas analyzing portion 19 is provided with a mass spectrometer besides NDIR, CLD, and FID for measuring concentrations of CO, $CO_2$, $NO_x$ and THC.

In the exhaust gas analyzing system with the above structure, the exhaust gas G from the engine 1 flows through the exhaust gas flow path 2 and is mixed with the trace gas T introduced into the exhaust gas flow path 2 through the trace gas introducing path 22. The flow rate of the trace gas T is controlled to be a constant value by the mass-flow controller 25 disposed in the trace gas introducing path 22. The flow rate at this time is monitored by the arithmetic controller 21.

The exhaust gas G and the trace gas T pass through the flowmeter 3 and a part of them flows into the sampling flow path 4 and the remainder is exhausted. Output from the flowmeter 3 is sent to the arithmetic controller 21.

The sample gas S and the trace gas T that have flown into the sampling flow path 4 are diluted with dilution gas D in the mini-diluter 6 and concentrations (concentrations in the bag) of respective components after dilution are obtained by NDIR, CLD, FID, the mass spectrometer, and the like in the gas analyzing portion 19 similarly to the exhaust gas analyzing system shown in Fig. 3. In this case, mass of the respective components (and also the trace gas T) can be also obtained in accordance with the above equation (1).

On the other hand, mass Mt of the trace gas T introduced into the exhaust gas flow path 2 through the trace gas introducing path 22 is given by the following equation (2).

$$M_t = C_t \times V_t \times \rho_t \qquad\qquad (2)$$

Where Ct represents a concentration of the trace gas T at introduction, $V_t$ the total introduced volume of the trace gas T, and $\rho_t$ density of the trace gas T.

In theory, $M_x$ given by the equation (1) and Mt given by the equation (2) should be the same value in respect of the trace gas T. Because Mt can be obtained relatively accurately, a difference between both the $M_x$ and Mt can be regarded as an indicator of correctness of the gas analysis for which the mini-diluter 6 is used.

As described above, in the exhaust gas analyzing system of the invention, the total mass of the trace gas T introduced into the exhaust gas G is known and by comparing the total mass of the trace gas T measured and calculated by using the mini-diluter method with the above known total mass it is possible to evaluate accuracy of the measurement itself. As a result, reliability of the exhaust gas analyzing system for which the mini-diluter method is used and which includes measurement of the flow rate of the exhaust gas is improved.

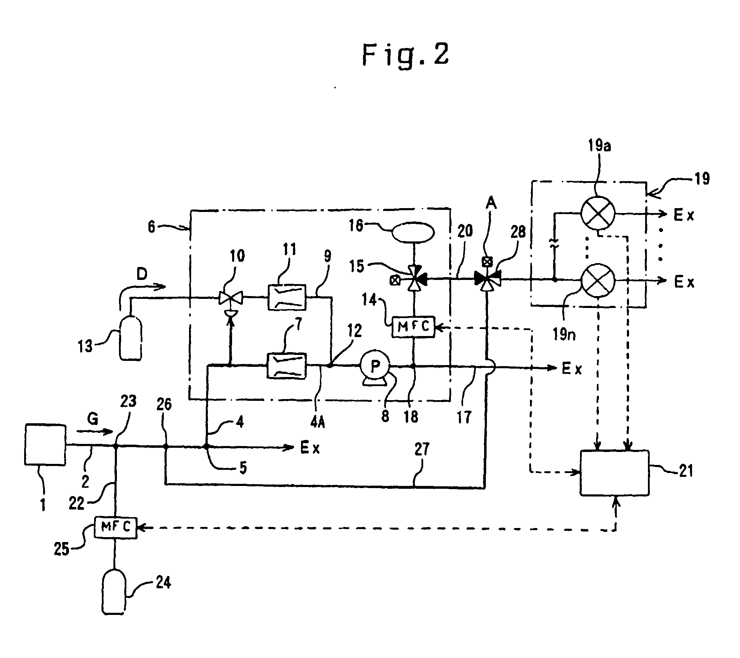

Fig. 2 shows a second embodiment of the invention. In this embodiment, the flow rate of gas (mixture of exhaust gas G and trace gas T) flowing through the exhaust gas flow path 2 is measured from a change of concentration of the trace gas T before mixing to the concentration after mixing. The flowmeter 3 is removed from the exhaust gas flow path 2, an end of a flow path 27 is connected to a point 26 between a connecting point 23 and a connecting point 5 of the exhaust gas flow path 2, and the other end of the flow path 27 is connected to a three-way solenoid valve 28 provided in a flow path 20.

In the structure shown in Fig. 2, sampling into the sample bag 16 is carried out while the concentration of the trace gas T in gas sampled from the connecting point 26 is measured in the gas analyzing portion 19. A flow rate of the exhaust gas G is calculated from the concentration of the trace gas after mixing in real time and fed back to the sampling flow rate into the sample bag 16. Sampling into the sample bag 16 ends and by analyzing components (including the trace gas T) in diluted gas obtained by the sampling it is possible to evaluate the measurement accuracy similar to the above first embodiment. In other words, because the trace gas T is used for measuring the flow rate of the exhaust gas G in the present embodiment, it is possible to omit the flowmeter 3 (in Fig. 1) for measuring the flow rate of the exhaust gas G.

The invention is not limited to the above respective embodiments and other chemically stable component such as $SF_6$ can be used as the trace gas T. If $SF_6$ is used as the trace gas T, an FTIR method gas analyzer including a Fourier transformation infrared spectrophotometer, for example, can be used as means for analyzing the concentration of $SF_6$, besides NDIR. Only by the FTIR method gas analyzer, CO, $CO_2$, NO, and $H_2O$ that are main components of the engine exhaust gas and $NO_2$, $N_2O$, $NH_3$ HCHO, and $CH_4$ of great interest can be measured simultaneously and the structure of the gas analyzing portion 19 can be simplified.

The structure for defining the flow rate in the mini-diluter 6 is not limited to the CFV; a flow rate controller such as mass-flow controller can be used, for example.

As described above, in the exhaust gas analyzing system of the invention, because measurement accuracy of the exhaust gas analyzing system when the mini-diluter method is used can be evaluated by the system itself, reliability of the exhaust gas analyzing system for which the mini-diluter method is used and which includes measurement of the flow rate of the exhaust gas is significantly improved.

**Claims**

1. An exhaust gas analyzing system comprising:

   - a sampling flow path (4) being connected at a connecting point (5) to an exhaust gas flow path (2) being connected to an engine (1) for sampling a part of said exhaust gas (G),
   - a dilution gas flow path (9) being connected in parallel to said sampling flow path (4) for diluting said exhaust gas (G) with dilution gas.
   - a sample bag (16) for reserving a part of said diluted exhaust gas being connected to said sampling flow path (4) downstream said dilution gas flow path (9) through a mass-flow controller (14) for controlling a flow rate of said diluted exhaust gas,
   - a gas analyzing portion (19) being connected to said sample bag (16) for analyzing said diluted exhaust gas in said sample bag
   - an arithmetic controller (21) being electrically connected to said mass-flow controller (14) for controlling the flow rate of said mass-flow controller (14) proportionally to that of said exhaust gas, at **characterized in that** it further comprises:

      a trace gas introducing path (22) for introducing of trace gas (T) with a known concentration into said exhaust gas flow path (2), said trace gas introducing path (22) being connected to a trace gas container (24) and being connected to said exhaust gas flow path at a connecting point (23) on an upstream side from the connecting point (5) between said exhaust gas flow path (2) and said sampling flow path (4), said trace gas introducing path (22) being further provided with a mass-flow controller (25) for measuring and controlling a flow rate of the trace gas (T), said mass flow controller (25) being electrically connected to said arithmetic controller (21) for providing said measured flow rate of said trace gas thereto,

   wherein the arithmetic controller (21) is comprising means for calculating the total mass of said trace gas (T) from a result of an analysis of said trace gas in said gas analysis portion (19) and comparing said calculated total mass of said trace gas (T) with the known total mass of said introduced trace gas to evaluate the accuracy of said analysing of said diluted exhaust gas.

2. A method of analyzing exhaust gas in an exhaust gas analyzing system, comprising a sampling flow path (4) being connected at a connecting point (5) to an exhaust gas flow path (2) being connected to an engine (1), said method comprising the steps of:

   - sampling a part of exhaust gast (G) exhausted from an engine (1) that flows through said exhaust gas flow path using said sampling flow path;
   - diluting said sampled exhaust gas with dilution gas introduced through a dilution gas flow path (9) connected in parallel to said sampling flow path,
   - reserving a part of said diluted exhaust gas in a sample bag (16) provided through a mass-flow controller (14).
   - determining a flow rate of said exhaust gas for controlling flow rate of

   said mass-flow controller (14) proportionally to that of said exhaust gas (G),

- analyzing said diluted exhaust gas in said sample bag in a gas analyzing portion (19) using said determined flow rate of said exhaust gas,
- introducing trace gas (T) with a known concentration into said exhaust gas flow path (2) at a connecting point (23) on an upstream side from the connecting point (5) between said exhaust gas flow path (2) and said sampling flow path (4) while monitoring a flow rate of said trace gas,
- analyzing diluted trace gas in said gas analyzing portion (19) and calculating the total mass of said trace gas from a result of said analysis,
- comparing said calculated total mass of said trace gas with known total mass of said introduced trace gas to evaluate the accuracy of said analyzing of said diluted exhaust gas,

wherein said flow rate of said exhaust gas is determined by monitoring concentration of said trace gas in gas sampled from a connecting point (26) between the connecting point (23) and the connecting point (5).

**Patentansprüche**

1. System zur Abgasanalyse, umfassend:

   - einen Probenahmeflusspfad (4), der bei einem Verbindungspunkt (5) mit einem Abgasflusspfad (2), verbunden mit einem Motor bzw. Antrieb (1), zur Probenahme eines Teils des Abgases (G), verbunden ist;
   - einen Verdünnungsgasflusspfad (9), der parallel mit dem Probenahmeflusspfad (4) zur Verdünnung des Abgases (G) mit Verdünnungsgas verbunden ist;
   - einen Probebehälter (16) zum Zurückhalten eines Teils des verdünnten Abgases, stromabwärts des Verdünnungsgasflusspfades (9) mit dem Probenahmeflusspfad (4) durch ein Masseflusssteuergerät (14) zur Steuerung der Flussgeschwindigkeit des verdünnten Abgases verbunden;
   - einen Gasanalyseabschnitt (19), der mit dem Probenbehälter (16) verbunden ist, zum Analysieren des verdünnten Abgases im Probenbehälter;
   - ein arithmetisches Steuergerät (21), das mit dem Masseflusssteuergerät (14) elektrisch verbunden ist, um die Flussgeschwindigkeit des Masseflusssteuergeräts (14) proportional zu derjenigen des Abgases zu steuern,

   **dadurch gekennzeichnet, dass** dieses weiterhin umfasst:

   einen Spurengaseinführpfad (22) zum Einführen eines Spurengases (T) mit einer bekannten Konzentration in den Abgasflusspfad (2), wobei der Spurengaseinführpfad (22) mit einem Spurengasbehälter (24) verbunden ist und mit dem Abgasflusspfad bei einem Verbindungspunkt (23) auf der stromaufwärtigen Seite des Verbindungspunktes (5) zwischen dem Abgasflusspfad (2) und dem Probenahmeflusspfad (4) verbunden ist, wobei der Spurengaseinführpfad (22) weiterhin mit einem Masseflusssteuergerät (25) zum Messen und Steuern der Flussgeschwindigkeit des Spurengases (T) vorgesehen ist, wobei das Masseflusssteuergerät (25) mit dem arithmetischen Steuergerät (21) elektrisch verbunden ist, um diesem die gemessene Flussgeschwindigkeit des Spurengases bereitzustellen, wobei das arithmetische Steuergerät (21) Mittel zum Berechnen der gesamten Masse des Spurengases (T) aus dem Ergebnis einer Analyse des Spurengases im Gasanalyseabschnitt (19) aufweist, und die berechnete Gesamtmasse des Spurengases (T) mit der bekannten Gesamtmasse des eingeführten Spurengases vergleicht, um die Genauigkeit der Analyse des verdünnten Abgases zu beurteilen.

2. Verfahren zur Analyse von Abgas in einem System zur Abgasanalyse, umfassend einen Probenahmeflusspfad (4), der bei einem Verbindungspunkt (5) mit einem Abgasflusspfad (2), verbunden mit einem Motor bzw. Antrieb (1), verbunden ist, wobei das Verfahren die Schritte umfasst:

   - Probenahme eines Teils des Abgases (G), das von einem Motor bzw. Antrieb (1) ausgestossen wird, das durch den Abgasflusspfad fließt, unter Verwendung des Probenahmeflusspfades;
   - Verdünnen des als Probe genommenen Abgases mit Verdünnungsgas, das durch einen Verdünnungsgasflusspfad (9) eingeführt wird, der parallel mit dem Probenahmeflusspfad verbunden ist;
   - Zurückhalten eines Teils des verdünnten Abgases in einem Probebehälter (16), bereitgestellt durch mit einem Masseflusssteuergerät (14);
   - Bestimmen der Flussgeschwindigkeit des Abgases zum Steuern der Flussgeschwindigkeit des Masseflusssteuergeräts (14) proportional zu derjenigen des Abgases (G);
   - Analysieren des verdünnten Abgases im Probebehälter in einem Gasanalyseabschnitt (19) unter Verwendung der bestimmten Flussgeschwindigkeit des Abgases;

- Einführen von Spurengas (T) mit einer bekannten Konzentration in den Abgasflusspfad (2) bei einem Verbindungspunkt (23) auf der stromaufwärtigen Seite des Verbindungspunktes (5) zwischen dem Abgasflusspfad (2) und dem Probenahmeflusspfad (4), während die Flussgeschwindigkeit des Spurengases überwacht wird;
- Analysieren des verdünnten Spurengases im Gasanalyseabschnitt (19) und Berechnen der Gesamtmasse des Spurengases aus dem Ergebnis der Analyse;
- Vergleichen der berechneten Gesamtmasse des Spurengases mit der bekannten Gesamtmasse des eingeführten Spurengases, um die Genauigkeit der Analyse des verdünnten Abgases zu beurteilen;

wobei die Flussgeschwindigkeit des Abgases durch Überwachen der Konzentration des Spurengases im Gas, das als Probe an dem Verbindungspunkt (26) zwischen dem Verbindungspunkt (23) und dem Verbindungspunkt (5) entnommen wird, bestimmt wird.

**Revendications**

1. Système d'analyse de gaz d'échappement comprenant :

un trajet d'écoulement d'échantillonnage (4) relié, au niveau d'un point de liaison (5), à un trajet d'écoulement de gaz d'échappement (2) lui-même relié à un moteur (1) pour échantillonner une partie dudit gaz d'échappement (G),
un trajet d'écoulement de gaz de dilution (9) relié en parallèle audit trajet d'écoulement d'échantillonnage (4) pour diluer ledit gaz d'échappement (G) avec un gaz de dilution,
un sac à échantillons (16) pour réserver une partie dudit gaz d'échappement dilué relié audit trajet d'écoulement d'échantillonnage (4) en aval dudit trajet d'écoulement de gaz de dilution (9) par l'intermédiaire d'un régulateur de débit massique (14) pour réguler un débit dudit gaz d'échappement dilué,
une partie d'analyse de gaz (19) reliée audit sac à échantillons (16) pour analyser ledit gaz d'échappement dilué dans ledit sac à échantillons,
un dispositif de commande arithmétique (21) relié électriquement audit régulateur de débit massique (14) pour réguler le débit dudit régulateur de débit massique (14), **caractérisé en ce qu'**il comprend en outre :

un trajet d'introduction de gaz à l'état de traces (22) pour introduire du gaz à l'état de traces (T) selon une concentration connue dans ledit trajet d'écoulement de gaz d'échappement (2), ledit trajet d'introduction de gaz à l'état de traces (22) étant relié à un contenant de gaz à l'état de traces (24) et étant relié audit trajet d'écoulement de gaz d'échappement au niveau d'un point de liaison (23) sur un côté amont par rapport au point de liaison (5) entre ledit trajet d'écoulement de gaz d'échappement (2) et ledit trajet d'écoulement d'échantillonnage (4), ledit trajet d'introduction de gaz à l'état de traces (22) étant en outre doté d'un régulateur de débit massique (25) pour mesurer et réguler un débit du gaz à l'état de traces (T), ledit régulateur de débit massique (25) étant relié électriquement audit dispositif de commande arithmétique (21) pour fournir ledit débit mesuré dudit gaz à l'état de traces à ce dernier, le dispositif de commande arithmétique (21) comprenant des moyens pour calculer le débit massique total dudit gaz à l'état de traces (T) à partir d'un résultat d'une analyse dudit gaz à l'état de traces dans ladite partie d'analyse de gaz (19) et pour comparer ledit débit massique total calculé dudit gaz à l'état de traces (T) au débit massique total connu dudit gaz à l'état de traces introduit pour évaluer l'exactitude de ladite analyse dudit gaz d'échappement dilué.

2. Procédé d'analyse de gaz d'échappement dans un système d'analyse de gaz d'échappement, comprenant un trajet d'écoulement d'échantillonnage (4) relié, au niveau d'un point de liaison (5), à un trajet d'écoulement de gaz d'échappement (2) lui-même relié à un moteur (1), ledit procédé comprenant les étapes consistant à :

échantillonner une partie du gaz d'échappement (G) s'échappant d'un moteur (1) qui circule à travers ledit trajet d'écoulement de gaz d'échappement à l'aide d'un trajet d'écoulement d'échantillonnage,
diluer ledit gaz d'échappement échantillonné avec un gaz de dilution introduit à travers un travers un trajet d'écoulement de gaz de dilution (9) relié en parallèle audit trajet d'écoulement d'échantillonnage,
réserver une partie dudit gaz d'échappement dilué dans un sac à échantillons (16) fournie par l'intermédiaire d'un régulateur de débit massique (14),
déterminer un débit dudit gaz d'échappement pour réguler le débit dudit régulateur de débit massique (14) proportionnellement à celui du gaz d'échappement (G),
analyser ledit gaz d'échappement dilué dans ledit sac à échantillons dans une partie d'analyse de gaz (19) à l'aide dudit débit de gaz d'échappement déterminé,

introduire un gaz à l'état de traces (T) selon une concentration connue dans ledit trajet d'écoulement de gaz d'échappement (2), au niveau d'un point de liaison (23), sur un côté amont par rapport au point de liaison (5) entre ledit trajet d'écoulement de gaz d'échappement (2) et ledit trajet d'écoulement d'échantillonnage (4) tout en contrôlant un débit dudit gaz à l'état de traces,

analyser le gaz à l'état de traces dilué dans ladite partie d'analyse de gaz (19) et calculer le débit massique total de gaz à l'état de traces à partir du résultat de ladite analyse,

comparer ledit débit massique total calculé dudit gaz à l'état de traces à un débit massique connu dudit gaz à l'état de traces introduit pour évaluer l'exactitude de l'analyse dudit gaz d'échappement dilué,

le débit dudit gaz d'échappement étant déterminé en contrôlant la concentration dudit gaz à l'état de traces dans du gaz échantillonné à partir d'un point de liaison (26) entre le point de liaison (23) et le point de connexion (5).

Fig. 1

Fig.2

# Fig.3

EP 1 106 983 B1

**REFERENCES CITED IN THE DESCRIPTION**

**Patent documents cited in the description**

- US 5968542 A

- US 4928015 A